# EUROPEAN PATENT SPECIFICATION

(11) **EP 3 454 962 B1**
(45) Date of publication and mention of the grant of the patent: **01.09.2021**
(21) Application number: 17725090.9
(22) Date of filing: 11.05.2017
(51) Int. Cl.: G01F 1/66, G01F 1/74, B01D 17/12, G01N 33/18, G01N 33/28

(54) **SYSTEM AND METHOD FOR FLUID INTERFACE IDENTIFICATION**
SYSTEM UND VERFAHREN ZUR IDENTIFIZIERUNG VON FLÜSSIGKEITSGRENZFLÄCHEN
SYSTÈME ET PROCÉDÉ D'IDENTIFICATION D'INTERFACE FLUIDIQUE

(30) Priority: 11.05.2016 US 201662334531 P; 10.05.2017 US 201715591254
(43) Date of publication of application: 20.03.2019
(73) Proprietor: General Electric Company, Schenectady, NY 12345 (US)
(72) Inventor: AO, Xiaolei, Shirley, Billerica, MA 01821 (US); GONG, Zheng, Billerica, MA 01821-4111 (US); WANG, Ji-yong, Billerica, MA 01821-4111 (US); MICHALOWSKI, Thomas, Edward, Billerica, MA 01821-4111 (US)
(74) Representative: BRP Renaud & Partner mbB Rechtsanwälte Patentanwälte Steuerberater
(86) International application number: PCT/US2017/032302
(87) International publication number: WO 2017/197209

(56) References cited:
- EP-A1- 2 211 150
- US-A- 5 741 980
- US-A1- 2009 281 671
- US-A1- 2012 017 998
- US-A1- 2013 082 010
- US-B1- 6 209 388

## Description

### BACKGROUND

The subject matter disclosed herein relates to a fluid interface identification process and, more particularly, to identification of a fluid interface in a conduit.

US 2009/281671 A1 discloses generating an alarm signal when the flow rate in a conduit indicates that a bubble is present in the conduit, and EP 2 211 150 A1 discloses an ultrasonic flowmeter determining a liquid-liquid interface flowing in the conduit.

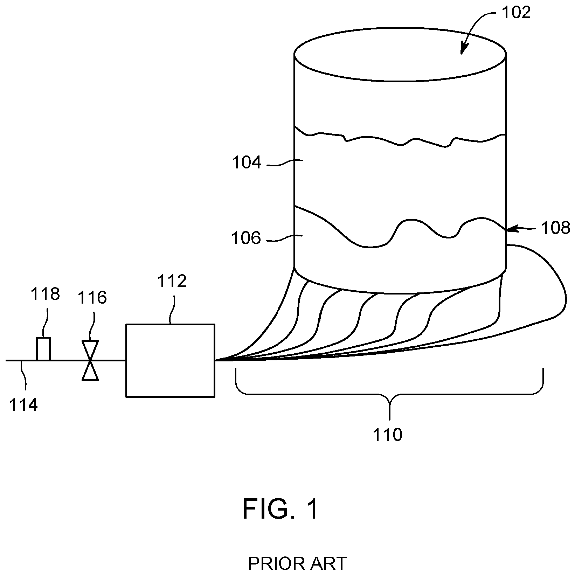

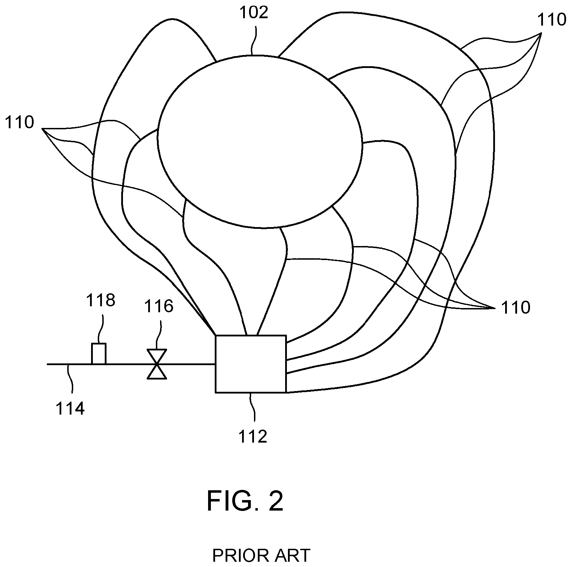

Refined chemical products, such as crude oil, may be stored in large tanks between process and transportation. Water can be present in crude or refined hydrocarbon liquid products and, in some cases, needs to be removed economically. Currently, dewatering (fluid separation) processes can be labor intensive and unsafe. As illustrated by FIGs. 1 and 2, a chemical storage tank 102 can include oil 104 and water 106, which separates due to the characteristics of the oil 104 and water 106, resulting in a water/oil interface 108. The chemical storage tank 102 typically includes a plurality of water draining pipes 110 positioned near the bottom of the tank 102 and equally spaced around the circumference of the tank 102. In some current applications, draining water is a manual process without a real time monitoring system to establish a stop time. In the illustrated tank 102, a small observation tank 112 can be positioned at the joining point of the draining pipes 110 before the common draining line 114. The observation tank 112 can be opened by an operator to visually observe the change of flow from water to crude oil when the draining valve 116 is open, signaling the operator to close the draining valve 116 and stop draining the water 106. This process can be time consuming, inefficient, and unsafe to operators.

In addition to visual observation, the speed of sound of fluid in the draining line 114 can be measured by a flow meter 118 to determine if the speed of sound indicates flow of water. If the speed of sound indicates the flow of a liquid other than water, the draining valve 116 can be closed. However, at certain range of temperatures, the speed of sound of oil and water is very close, which can make it difficult to distinguish between the two.

### SUMMARY

The present invention is defined in the accompanying claims.

A system and method for identifying a fluid interface are disclosed. The system and method may address one or more of the problems noted above. For instance, an accurate identification of the fluid may require a temperature compensation combined with pre-sampling result of the fluids. At certain temperatures, when the two speed of sounds are identical, there may be no way to determine if water or oil is flowing by this technique.

An advantage that may be realized in the practice of some disclosed embodiments is simplification of systems and methods for identifying fluids and fluid transitions in a conduit.

The above embodiments are exemplary only. Other embodiments are within the scope of the disclosed subject matter.

### BRIEF DESCRIPTION OF THE DRAWINGS

So that the manner in which the features of the invention can be understood, a detailed description of the invention may be made by reference to certain embodiments, some of which are illustrated in the accompanying drawings. It is to be noted, however, that the drawings illustrate only certain embodiments of this invention and are therefore not to be considered limiting of its scope, for the scope of the disclosed subject matter encompasses other embodiments as well. The drawings are not necessarily to scale, emphasis generally being placed upon illustrating the features of certain embodiments of the invention. In the drawings, like numerals are used to indicate like parts throughout the various views.
FIG. 1 is an illustration of an embodiment of a prior art chemical storage tank;
FIG. 2 is a top view of an illustration of the prior art chemical storage tank of FIG. 1;
FIG. 3 is a diagram of an embodiment of system for identifying a liquid-liquid interface;
FIG. 4A is an illustration of an embodiment of a two-transducer, angled signal, clamp-on flow meter;
FIG. 4B is an illustration of another embodiment of a two-transducer, angled signal, clamp-on flow meter;
FIG. 4C is an illustration of an embodiment of a two-transducer, vertical signal, clamp-on flow meter;
FIG. 4D is an illustration of an embodiment of a single transducer, vertical signal, clamp-on flow meter;
FIG. 5A is an illustration of an embodiment of a two-transducer, angled signal, wetted flow meter;
FIG. 5B is an illustration of another embodiment of a two-transducer, angled signal, wetted flow meter;
FIG. 5C is an illustration of an embodiment of a two-transducer, vertical signal, wetted flow meter,
FIG. 5D is an illustration of an embodiment of a single transducer, vertical signal, wetted flow meter;
FIG. 6 is a flow diagram illustrating an embodiment of a method of identifying a liquid-liquid interface;
FIG. 7 is an illustration of a three phase system;
FIG. 8 is a graph illustrating a standard deviation of an embodiment of an acoustic signal amplitude;
FIG. 9 is a graph illustrating a speed of sound measurement and standard deviation of the speed of sound;
FIG. 10 is a series of graphs illustrating an example of the amplitude of five acoustic signals measured in a first liquid,
FIG. 11 is a series of graphs illustrating an example of the amplitude of the five acoustic signals of FIG. 10 measured at the liquid-liquid interface; and
FIG. 12 is a series of graphs illustrating an example of the amplitude of the five acoustic signals of FIGs. 10-11 measured in a second liquid.

### DETAILED DESCRIPTION

Embodiments of the disclosed subject matter are directed to identifying liquid-liquid interfaces. In particular, embodiments are directed to identifying liquid-liquid interfaces in liquids flowing in a conduit. In this embodiment, an acoustic signal can be applied to a conduit through which a liquid is flowing and is used to measure the speed of sound within the liquid flowing in the conduit. The characteristics of the acoustic signal are tracked and, when a change in the characteristics of the acoustic signal is identified, the presence of the liquid-liquid interface is identified and the type of liquid flowing in the draining line can be identified as having changed from a first liquid to a second liquid.

In an example, the method can be used for draining the water from a mixture of oil and water stored in a tank. In this example, a draining valve of a draining line or pipe can be opened and draining of the water begins. The acoustic signal can be applied to the draining line and, when a change in the characteristics of the acoustic signal is identified, the type of liquid flowing in the draining line is identified as having changed from water to oil and the draining valve is closed. While embodiments of some processes are described above, it is to be understood that additional embodiments are possible.

The system and method described herein can employ a temperature-independent analysis of the dynamics of an acoustic signal to determine when the emulsion point or interface between two liquids is present in a conduit. Because the analysis is temperature independent, the system and method of this process are simplified, improving accuracy and reducing the cost of the processes, such as dewatering processes. The system and method further includes measuring the signal strength or the transmitted acoustic signal amplitude and determining the standard deviation of the signal. When the standard deviation exceeds a predetermined threshold, emulsion of the two liquids is determined.

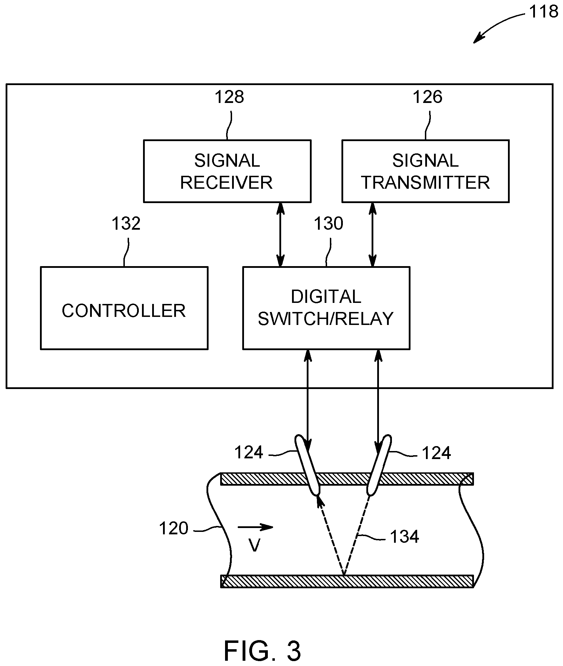

FIG. 3 is a diagram of an embodiment of a system for identifying the fluid interface in a conduit. In the illustrated embodiment, liquids, such as a first and second liquid, flowing V through the conduit 120. In this embodiment a first liquid flows through the conduit 120 first, followed by the second liquid, with a fluid interface being present between the first and second liquid. In this embodiment, an ultrasonic flow meter 118 is coupled to the conduit 120.

The ultrasonic flow meter 118 includes at least one ultrasonic transducer 124 coupled to the conduit 120. In the illustrated embodiment, two ultrasonic transducers 124 are coupled to the conduit 120. The at least one ultrasonic transducer 124 is configured to transmit an acoustic signal 134 into the liquid flowing V in the conduit 120 and receive reflected ultrasonic signals. A signal transmitter 126 can initiate transmission of the acoustic signal 134 and a signal receiver 128 receives the reflected signals from the ultrasonic transducers 124. A digital switch/relay 130 can couple the ultrasonic transducer(s) 124 to the signal transmitter 126 and signal receiver 128.

A controller 132 is configured to analyze the reflected signals. The controller 132 is configured to determine the speed of sound of the acoustic signal 134 based on the amplitude and transit time of the reflected signal and calculate the average and standard deviation of the signal amplitude, speed of sound, or a combination thereof. By comparing the calculated standard deviation to a predetermined threshold, the controller 132 identifies the presence of the liquid-liquid interface. When the standard deviation exceeds the predetermined threshold, liquid-liquid interface is identified as being present. The system can be used to determine a liquid-liquid interface between liquids having different specific gravities. For example, the system can be used to determine the liquid-liquid interface between oil and water or between a first and second oil, among other liquids.

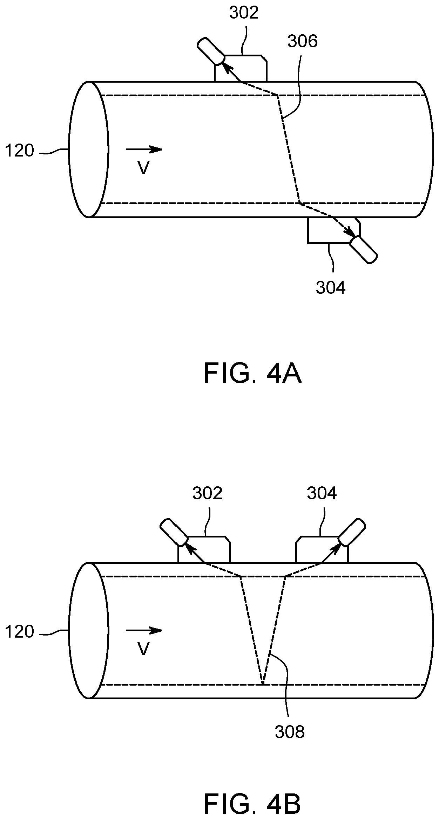

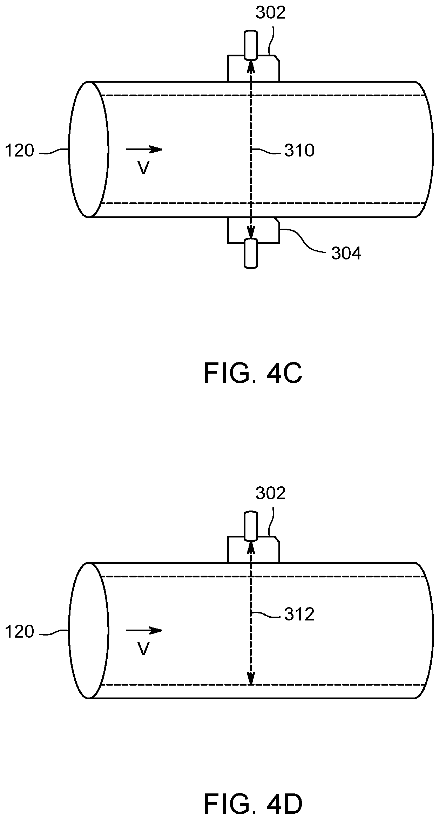

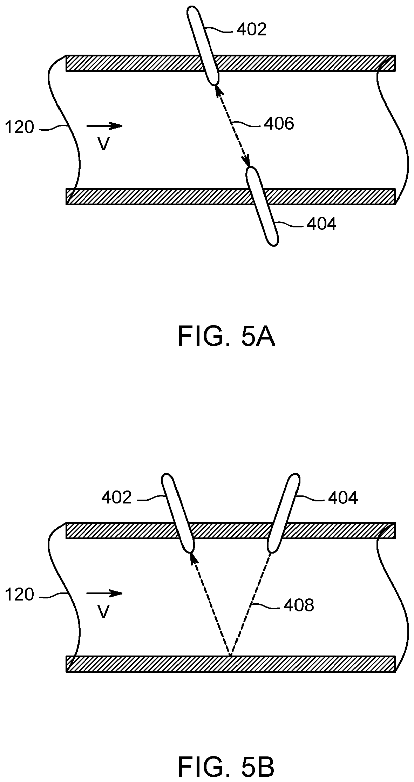

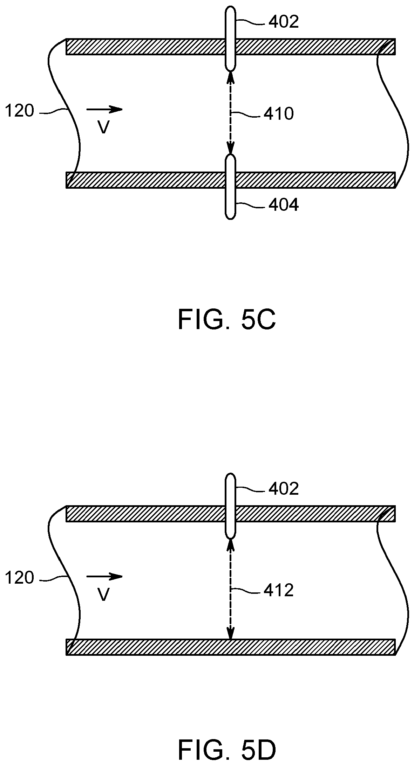

As illustrated by FIGs. 4A-4D, the flow meter 118 can be a clamp-on flow meter. As illustrated in FIGs. 4A-4B, the flow meter 118 can include two transducers 302, 304, which may be clamped to the conduit 120 that transmit and receive an angled acoustic signal 306, 308 through the liquid flowing V in the conduit 120. In another embodiment, illustrated in FIG. 4C, the flow meter 118 can include two transducers 302, 304, which can be clamped to the conduit 120 that transmit and receive a vertical acoustic signal 310 through the liquid flowing V in the conduit 120. In yet another embodiment, illustrated in FIG. 4D, the flow meter 118 can include a single transducer 302 that transmits and receives a vertical acoustic signal 312 through the liquid flowing V in the conduit 120. In another embodiment, as illustrated by FIGs. 5A-5D, the flow meter can be a wetted flow meter. As illustrated by FIGs. 5A-5B, the flow meter 118 can include two transducers 402, 404 in contact with the liquid flowing V through the conduit 120 to transmit and receive an angled acoustic signal 406, 408. In another embodiment, illustrated in FIG. 5C, the flow meter 118 can include two transducers 402, 404 that transmit and receive a vertical acoustic signal through the liquid flowing V in the conduit 120. In yet another embodiment, illustrated in FIG. 5D, the flow meter 118 can include a single transducer 402 that transmits and receives a vertical acoustic signal 412 through the fluid flowing V in the conduit 120.

FIGs. 1 and 2 illustrate an embodiment of an environment in which this system can be employed. As discussed above, FIGs. 1 and 2 illustrate a chemical storage tank 102 storing two liquids 104, 106, such as water 106 and oil 104. Each of these two liquids 104, 106 meet at an interface 108. A plurality of draining lines 110 are coupled to the tank 102 and join to form a common draining line or conduit 114. A draining valve 116 is positioned on the common draining line 114. In an embodiment, an observation tank 112 can be coupled to the common draining line 114.

In an embodiment, a flow meter 118 can be coupled to the common draining line 114. The flow meter 118 includes at least one transducer that transmits and receives an acoustic signal. The at least one transducer transmits an acoustic signal within any suitable frequency range, such as within a frequency range of about 50kHz to about 5MHz. In other embodiments, the frequency range may be less than about 50kHz to greater than 5MHz. In an embodiment, the acoustic signal can be an analog signal that is digitized to a digital signal. The acoustic signal is an ultrasonic signal. In an embodiment, the flow meter 118 includes at least two transducers.

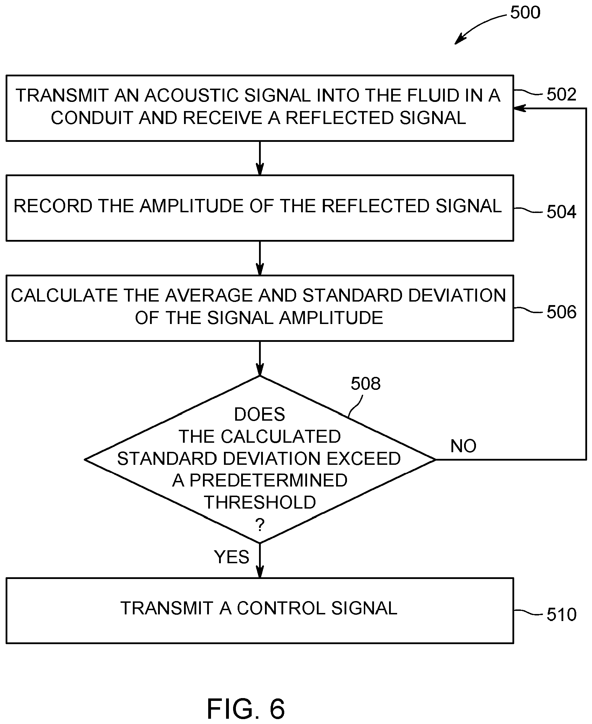

FIG. 6 is a flow chart illustrating an embodiment of a method 500 of identifying a fluid interface. In some embodiments, the steps may be performed in the order presented in FIG. 6 and/or as described herein. In other embodiments, one or more of the steps may be omitted, additional steps may be added, or the steps may be performed in a different order. The method 500 can be enacted in a conduit 120 having a flow meter 118 coupled thereto. The method can begin at block 502 when an acoustic signal is transmitted and received through the liquid flowing in the conduit 120. The flow meter 118 includes at least one transducer 124, such as an ultrasonic transducer, arranged in any suitable configuration, such as one of the configurations illustrated in FIGs. 4A-4D and 5A-5D. At block 504, the amplitude of the received acoustic signal is recorded. The transit time of the received acoustic signal is recorded and the speed of sound (SOS) of the fluid is determined based on the speed of sound. The amplitude and other information, such as SOS, can be stored, such as in the flow meter 118, and the signal amplitude and/or SOS can be displayed to an operator. In an embodiment, an acoustic signal can be transmitted into the liquid at regular time intervals.

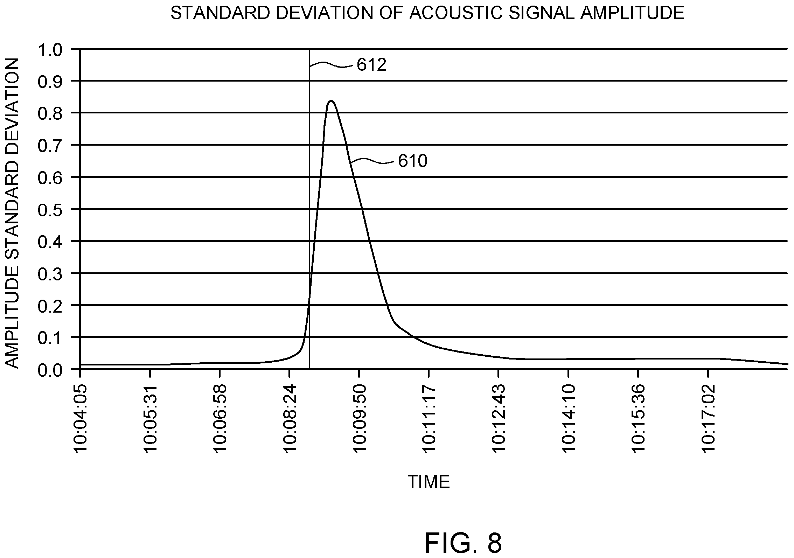

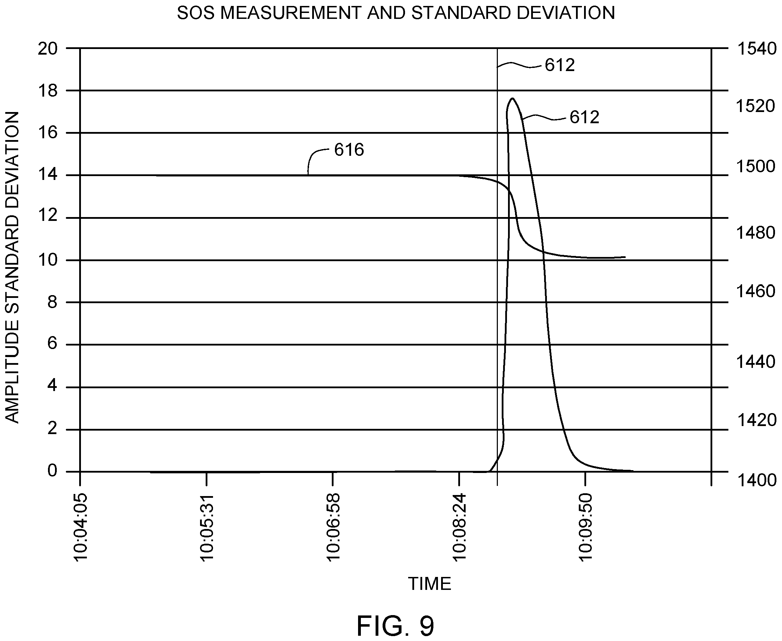

At block 506, the average amplitude or signal strength and the standard deviation the signal amplitude is determined. The standard deviation of the speed of sound is determined. In an example, the average is the n-point average, with n corresponding to a particular time, such as 1.5 seconds. An example of a determined standard deviation of amplitude 610 and the fluid transition interface 612 are illustrated in the graph of FIG. 8. In FIG. 9, the standard deviation 614 of speed of sound is displayed along with the determined speed of sound 616 and the fluid interface 612.

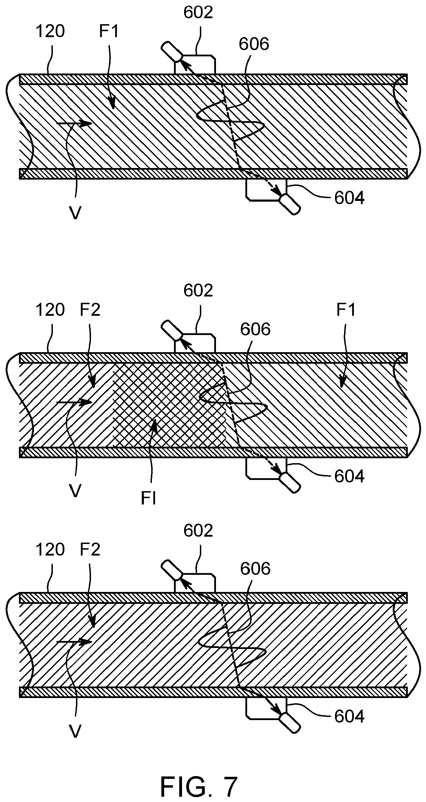

As illustrated in FIG. 7, the flow meter 118 is used to measure the speed of sound in a two-liquid, three phase system flowing in a conduit 120. As illustrated in FIG. 7, the three phase system includes a first liquid F1, a second liquid F2, and a fluid interface FI. For example, in a dewatering process, the liquids can be water and oil and the three phases can be water only, emulsion of water and oil, and oil only. The flow meter 118 includes at least one transducer 602, 604 that transmits and receives an acoustic signal 606 through the liquid flowing V in the conduit 120. In a dewatering process, the draining valve 116 (FIGs. 1 and 2) of a draining line 114 may be closed when the emulsion phase is determined to be flowing within the draining line 114.

Returning to FIG. 6, at block 508, it is determined if the calculated standard deviation exceeds a predetermined threshold. In an example, the predetermined threshold can be 0.5. If the calculated standard deviation does not exceed the predetermined threshold, the first phase, such as the water phase, is determined to be flowing within the draining line 114 and the method may return to block 502. If the calculated standard deviation does exceed the predetermined threshold, a phase other than the first phase, such as the second or third phase, e.g., emulsion or oil phases, are determined to be flowing within the draining line 114. At block 510, a control signal can be transmitted, such as a signal to close the draining valve 116. In an embodiment, the signal is transmitted to an operator to manually close the draining valve 116. In another embodiment, the signal initiates automatic closure of the draining valve 116. The signal amplitude and SOS, as well as their respective standard deviations, can be displayed. The signal amplitude, SOS, or a combination of signal amplitude and SOS is used to determine the presence of the phases.

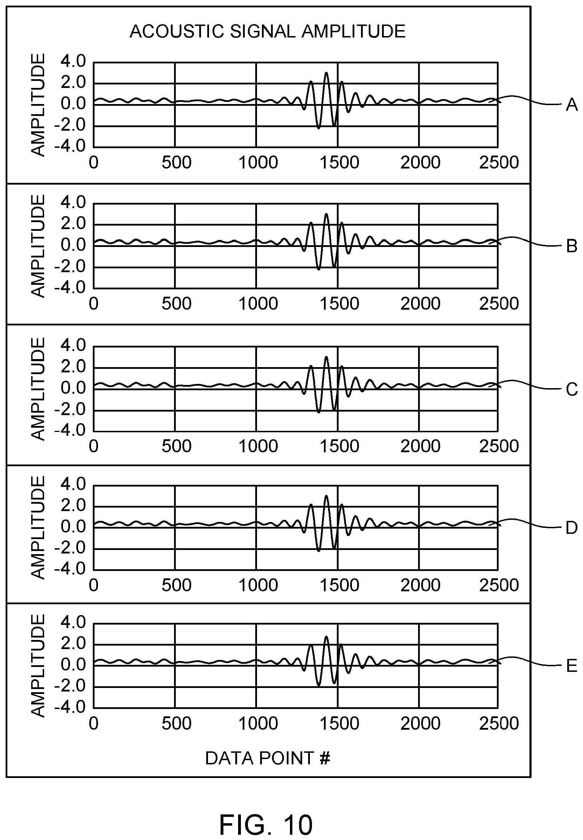

FIGs. 10-12 illustrate an example of signal amplitude from a two liquid, three phase system. FIG. 10 illustrates five acoustic signals measured in a first liquid, with the amplitude A, B, C, D, E of the five signals being measured at various time points. Amplitude A was measured at time T1, amplitude B was measured at time T2, amplitude C was measured at time T3, amplitude D was measured at time T4, and amplitude E was measured at time T5. The measured amplitudes A, B, C, D, E of the five signals are used to calculate standard deviation. As illustrated, in this first liquid, the amplitude undergoes a small fluctuation, which results in a standard deviation less than 0.15.

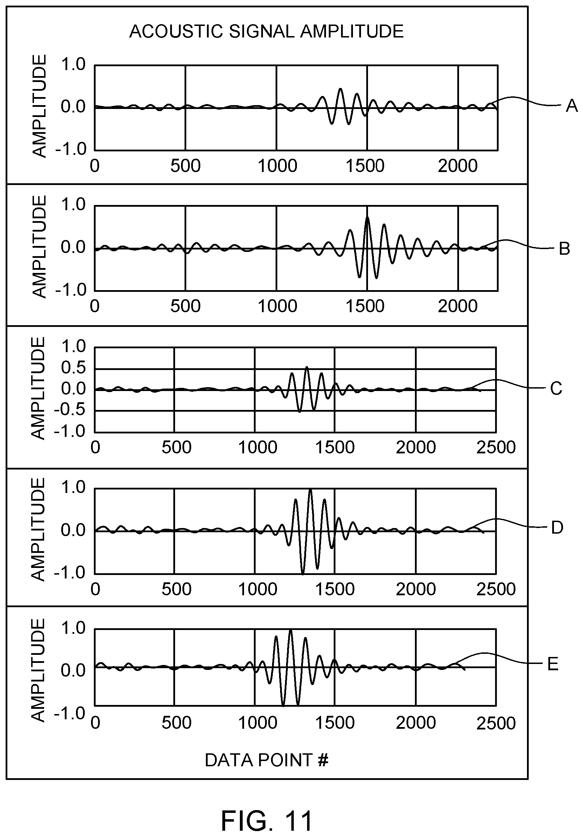

FIG. 11 illustrates the amplitudes A, B, C, D, E of the five signals as measured at the liquid transition zone. As illustrated in FIG. 11, at the liquid transition zone, the amplitudes undergo a large fluctuation, resulting in a standard deviation greater than 0.15.

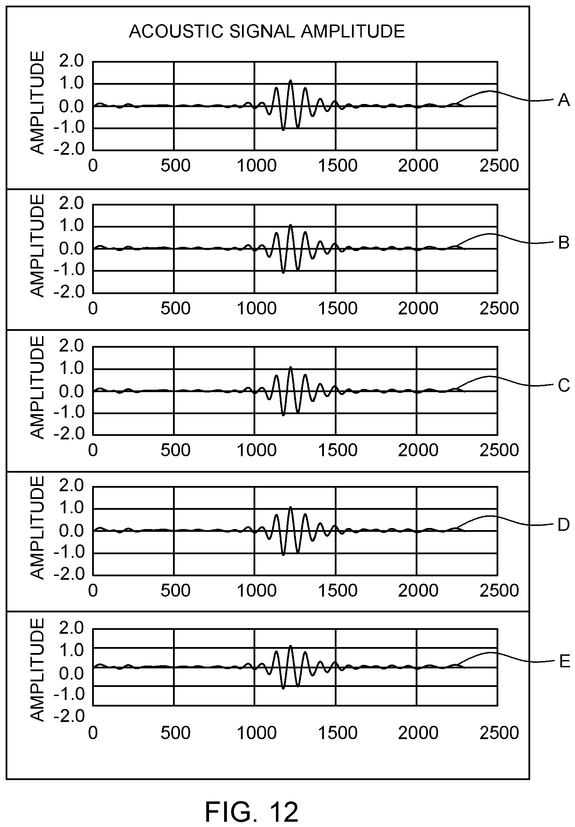

FIG. 12 illustrates the amplitudes A, B, C, D, E of the five signals as measured in the second liquid. As illustrated in FIG. 12 and similar to measurement in the first liquid, the measured amplitudes A, B, C, D, E undergo small fluctuations, again resulting in a standard deviation less than 0.15.

Possible advantages of the above described method and system include temperature independent identification of liquid interfaces and of separation of liquids.

While the present invention has been particularly shown and described with reference to certain exemplary embodiments, it will be understood by one skilled in the art that various changes in detail may be effected therein without departing from the scope of the invention that can be supported by the written description and drawings. Further, where exemplary embodiments are described with reference to a certain number of elements it will be understood that the exemplary embodiments can be practiced utilizing more than the certain number of elements.

The patentable scope of the invention is defined by the claims.

To the extent that the claims recite the phrase "at least one of' in reference to a plurality of elements, this recitation is intended to mean at least one or more of the listed elements, and is not limited to at least one of each element. For example, "at least one of an element A, element B, and element C," is intended to indicate element A alone, or element B alone, or element C alone, or any combination thereof. "At least one of element A, element B, and element C" is not intended to be limited to at least one of an element A, at least one of an element B, and at least one of an element C.

The subject matter described herein can be implemented in digital electronic circuitry, or in computer software, firmware, or hardware, including the structural means disclosed in this specification and structural equivalents thereof, or in combinations of them. The subject matter described herein can be implemented as one or more computer program products, such as one or more computer programs tangibly embodied in an information carrier (e.g., in a machine-readable storage device), or embodied in a propagated signal, for execution by, or to control the operation of, data processing apparatus (e.g., a programmable controller, a computer, or multiple computers). A computer program (also known as a program, software, software application, or code) can be written in any form of programming language, including compiled or interpreted languages, and it can be deployed in any form, including as a stand-alone program or as a module, component, subroutine, or other unit suitable for use in a computing environment. A computer program does not necessarily correspond to a file. A program can be stored in a portion of a file that holds other programs or data, in a single file dedicated to the program in question, or in multiple coordinated files (e.g., files that store one or more modules, sub-programs, or portions of code). A computer program can be deployed to be executed on one computer or on multiple computers at one site or distributed across multiple sites and interconnected by a communication network.

The processes and logic flows described in this specification, including the method steps of the subject matter described herein, can be performed by one or more programmable controllers executing one or more computer programs to perform functions of the subject matter described herein by operating on input data and generating output. The processes and logic flows can also be performed by, and apparatus of the subject matter described herein can be implemented as, special purpose logic circuitry, e.g., an FPGA (field programmable gate array) or an ASIC (application-specific integrated circuit).

Controllers suitable for the execution of a computer program include, by way of example, both general and special purpose microcontrollers, and any one or more controller of any kind of digital computer. Generally, a controller will receive instructions and data from a read-only memory or a random access memory or both. The essential elements of a computer are a controller for executing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. Information carriers suitable for embodying computer program instructions and data include all forms of non-volatile memory, including by way of example semiconductor memory devices, (e.g., EPROM, EEPROM, and flash memory devices); magnetic disks, (e.g., internal hard disks or removable disks); magneto-optical disks; and optical disks (e.g., CD and DVD disks). The controller and the memory can be supplemented by, or incorporated in, special purpose logic circuitry.

To provide for interaction with a user, the subject matter described herein can be implemented on a computer having a display device, e.g., a CRT (cathode ray tube) or LCD (liquid crystal display) monitor, for displaying information to the user and a keyboard and a pointing device, (e.g., a mouse or a trackball), by which the user can provide input to the computer. Other kinds of devices can be used to provide for interaction with a user as well. For example, feedback provided to the user can be any form of sensory feedback, (e.g., visual feedback, auditory feedback, or tactile feedback), and input from the user can be received in any form, including acoustic, speech, or tactile input.

The techniques described herein can be implemented using one or more modules. As used herein, the term "module" refers to computing software, firmware, hardware, and/or various combinations thereof. At a minimum, however, modules are not to be interpreted as software that is not implemented on hardware, firmware, or recorded on a non-transitory controller readable recordable storage medium (i.e., modules are not software *per se*). Indeed "module" is to be interpreted to always include at least some physical, non-transitory hardware such as a part of a controller or computer. Two different modules can share the same physical hardware (e.g., two different modules can use the same controller and network interface). The modules described herein can be combined, integrated, separated, and/or duplicated to support various applications. Also, a function described herein as being performed at a particular module can be performed at one or more other modules and/or by one or more other devices instead of or in addition to the function performed at the particular module. Further, the modules can be implemented across multiple devices and/or other components local or remote to one another. Additionally, the modules can be moved from one device and added to another device, and/or can be included in both devices.

The subject matter described herein can be implemented in a computing system that includes a back-end component (e.g., a data server), a middleware component (e.g., an application server), or a front-end component (e.g., a client computer having a graphical user interface or a web browser through which a user can interact with an implementation of the subject matter described herein), or any combination of such back-end, middleware, and front-end components. The components of the system can be interconnected by any form or medium of digital data communication, e.g., a communication network. Examples of communication networks include a local area network ("LAN") and a wide area network ("WAN"), e.g., the Internet.

## Claims

1. A method for identifying a liquid-liquid interface (FI) between a first liquid (F1) and a second liquid (F2) in a conduit (120) through which at least the first liquid (F1) and the second liquid (F2) are configured to pass, the conduit (120) having an ultrasonic flow meter (118) coupled thereto, the method comprising:
transmitting, via the ultrasonic flow meter (118), an acoustic signal into the conduit (120);
receiving, via the ultrasonic flow meter (118), a reflected acoustic signal;
determining, via a controller (132), an amplitude of the reflected acoustic signal;
**characterized by** calculating a standard deviation of the amplitude of the reflected acoustic signal measured at various time points;
determining if the standard deviation of the amplitude of the reflected acoustic signal exceeds a predetermined threshold of the amplitude of the reflected acoustic signal;
identifying the liquid-liquid interface in the conduit (120) when the standard deviation of the amplitude of the reflected acoustic signal exceeds the predetermined threshold of the amplitude of the reflected acoustic signal, wherein the liquid -liquid interface (FI) is identified independent of the temperature;
determining, via the controller (132), a speed of sound of the reflected acoustic signal based on transit time of the reflected acoustic signal;
calculating a standard deviation of the speed of sound of the reflected acoustic signal;
determining if the standard deviation of the speed of sound of the reflected acoustic signal exceeds a predetermined threshold of the speed of sound of the reflected acoustic signal; and
identifying the liquid-liquid interface in the conduit (120) when the standard deviation of the speed of sound of the reflected acoustic signal exceeds the predetermined threshold of the speed of sound of the reflected acoustic signal.

2. The method of claim 1, wherein the first liquid (F1) has a first specific gravity and the second liquid (F2) has a second specific gravity that is different from the first specific gravity.

3. The method of claim 2, wherein the first liquid (F1) is oil and the second liquid (F2) is water.

4. The method of claim 2, wherein the first liquid is a first oil and the second liquid is a second oil.

5. The method of any preceding claim, further comprising transmitting a signal to indicate identification of the liquid-liquid interface.

6. The method of any preceding claim, further comprising transmitting the acoustic signal when a liquid is flowing in the conduit (120).

7. The method of any preceding claim, wherein the conduit is a draining line of a chemical storage tank (120) and wherein the method further comprises transmitting a control signal to close the draining line when the liquid-liquid interface is identified.

8. The method of any preceding claim, further comprising determining, via the controller (132), a speed of sound of the reflected acoustic signal based on the amplitude and transit time of the reflected acoustic signal.

9. A system for identifying a liquid-liquid interface (FI) between a first liquid (F1) and a second liquid (F2) in a conduit (120) through which at least the first liquid (F1) and the second liquid (F2) are configured to pass, comprising:
an ultrasonic flow meter (118) coupled to the conduit (120), the flow meter comprising:
at least one ultrasonic transducer (124) coupled to the conduit (120) and configured to transmit an ultrasonic signal into the conduit (120) and receive a reflected ultrasonic signal; and
a controller (132) coupled to the at least one ultrasonic transducer (124) and configured to:
determine an amplitude of the reflected ultrasonic signal;
**characterized by** the controller (132) being configured to: calculate a standard deviation of the amplitude of the reflected ultrasonic signal measured at various points;
determine if the standard deviation of the amplitude of the reflected ultrasonic signal exceeds a predetermine threshold of the amplitude of the reflected ultrasonic signal;
identify the liquid-liquid interface in the conduit (120) when the standard deviation of the amplitude of the reflected ultrasonic signal exceeds the predetermined threshold of the amplitude of the reflected ultrasonic signal, wherein the liquid-liquid interface (FI) is identified independent of the temperature;
determine, via the controller, a speed of sound of the reflected ultrasonic signal based on transit time of the reflected ultrasonic signal;
calculate a standard deviation of the speed of sound of the reflected ultrasonic signal;
determine if the standard deviation of the speed of sound of the reflected ultrasonic signal exceeds a predetermined threshold of the speed of sound of the reflected ultrasonic signal; and
identify the liquid-liquid interface in the conduit (120) when the standard deviation of the speed of sound of the reflected ultrasonic signal exceeds the predetermined threshold of the speed of sound of the reflected ultrasonic signal.

10. The system of claim 9, wherein the conduit (120) is a draining line coupled to a chemical storage tank (102), the draining line comprising a draining valve (116).

11. The system of claim 10, wherein the controller (132) is further configured to transmit a signal to close the draining valve (116) when the liquid-liquid interface is identified in the conduit (120).

12. The system of any of claims 9 to 11, wherein the first liquid has a first specific gravity and the second liquid has a second specific gravity different from the first specific gravity.

13. The system of claim 12, wherein the first liquid is oil and the second liquid is water.

## Patentansprüche

1. Verfahren zum Identifizieren einer Flüssig-Flüssig-Grenzfläche (FI) zwischen einer ersten Flüssigkeit (F1) und einer zweiten Flüssigkeit (F2) in einer Leitung (120), durch die mindestens die erste Flüssigkeit (F1) und die zweite Flüssigkeit (F2) geleitet werden, wobei die Leitung (120) einen daran gekoppelten Ultraschalldurchflussmesser (118) aufweist, wobei das Verfahren Folgendes umfasst:
Übertragen eines akustischen Signals in die Leitung (120) über den Ultraschalldurchflussmesser (118);
Empfangen eines reflektierten akustischen Signals über den Ultraschalldurchflussmesser (118);
Bestimmen einer Amplitude des reflektierten akustischen Signals über eine Steuerung (132);
**gekennzeichnet durch** Berechnen einer Standardabweichung der Amplitude des reflektierten akustischen Signals, die zu verschiedenen Zeitpunkten gemessen wird;
Bestimmen, ob die Standardabweichung der Amplitude des reflektierten akustischen Signals einen vorbestimmten Schwellenwert der Amplitude des reflektierten akustischen Signals überschreitet;
Identifizieren der Flüssig-Flüssig-Grenzfläche in der Leitung (120), wenn die Standardabweichung der Amplitude des reflektierten akustischen Signals den vorbestimmten Schwellenwert der Amplitude des reflektierten akustischen Signals überschreitet, wobei die Flüssig-Flüssig-Grenzfläche (FI) unabhängig von der Temperatur identifiziert wird;
Bestimmen einer Schallgeschwindigkeit des reflektierten akustischen Signals basierend auf der Laufzeit des reflektierten akustischen Signals über die Steuerung (132);
Berechnen einer Standardabweichung der Schallgeschwindigkeit des reflektierten akustischen Signals;
Bestimmen, ob die Standardabweichung der Schallgeschwindigkeit des reflektierten akustischen Signals einen vorbestimmten Schwellenwert der Schallgeschwindigkeit des reflektierten akustischen Signals überschreitet; und
Identifizieren der Flüssig-Flüssig-Grenzfläche in der Leitung (120), wenn die Standardabweichung der Schallgeschwindigkeit des reflektierten akustischen Signals den vorbestimmten Schwellenwert der Schallgeschwindigkeit des reflektierten akustischen Signals überschreitet.

2. Verfahren nach Anspruch 1, wobei die erste Flüssigkeit (F1) ein erstes spezifisches Gewicht aufweist und die zweite Flüssigkeit (F2) ein zweites spezifisches Gewicht aufweist, das sich von dem ersten spezifischen Gewicht unterscheidet.

3. Verfahren nach Anspruch 2, wobei die erste Flüssigkeit (F1) Öl ist und die zweite Flüssigkeit (F2) Wasser ist.

4. Verfahren nach Anspruch 2, wobei die erste Flüssigkeit ein erstes Öl ist und die zweite Flüssigkeit ein zweites Öl ist.

5. Verfahren nach einem der vorstehenden Ansprüche, ferner umfassend das Übertragen eines Signals, um die Identifikation der Flüssig-Flüssig-Grenzfläche anzugeben.

6. Verfahren nach einem der vorstehenden Ansprüche, ferner umfassend das Übertragen des akustischen Signals, wenn eine Flüssigkeit in der Leitung (120) fließt.

7. Verfahren nach einem der vorstehenden Ansprüche, wobei die Leitung eine Ablassleitung eines Chemikalienlagertanks (120) ist und wobei das Verfahren ferner das Übertragen eines Steuersignals zum Schließen der Ablassleitung umfasst, wenn die Flüssig-Flüssig-Grenzfläche identifiziert wird.

8. Verfahren nach einem der vorstehenden Ansprüche, ferner umfassend das Bestimmen einer Schallgeschwindigkeit des reflektierten akustischen Signals basierend auf der Amplitude und Laufzeit des reflektierten akustischen Signals über die Steuerung (132).

9. System zum Identifizieren einer Flüssig-Flüssig-Grenzfläche (FI) zwischen einer ersten Flüssigkeit (F1) und einer zweiten Flüssigkeit (F2) in einer Leitung (120), durch die mindestens die erste Flüssigkeit (F1) und die zweite Flüssigkeit (F2) geleitet werden, umfassend:
einen Ultraschalldurchflussmesser (118), der mit der Leitung (120) gekoppelt ist, wobei der Durchflussmesser Folgendes umfasst:
mindestens einen Ultraschallwandler (124), der mit der Leitung (120) gekoppelt ist und konfiguriert ist, um ein Ultraschallsignal in die Leitung (120) zu senden und ein reflektiertes Ultraschallsignal zu empfangen; und
eine Steuerung (132), die mit dem mindestens einen Ultraschallwandler (124) gekoppelt ist und konfiguriert ist zum:
Bestimmen einer Amplitude des reflektierten Ultraschallsignals;
**dadurch gekennzeichnet, dass** die Steuerung (132) konfiguriert ist zum
Berechnen einer Standardabweichung der Amplitude des reflektierten Ultraschallsignals, die an verschiedenen Punkten gemessen wird;
Bestimmen, ob die Standardabweichung der Amplitude des reflektierten Ultraschallsignals einen vorbestimmten Schwellenwert der Amplitude des reflektierten Ultraschallsignals überschreitet;
Identifizieren der Flüssig-Flüssig-Grenzfläche in der Leitung (120), wenn die Standardabweichung der Amplitude des reflektierten Ultraschallsignals den vorbestimmten Schwellenwert der Amplitude des reflektierten Ultraschallsignals überschreitet, wobei die Flüssig-Flüssig-Grenzfläche (FI) unabhängig von der Temperatur identifiziert wird;
Bestimmen einer Schallgeschwindigkeit des reflektierten Ultraschallsignals basierend auf der Laufzeit des reflektierten Ultraschallsignals über die Steuerung;
Berechnen einer Standardabweichung der Schallgeschwindigkeit des reflektierten Ultraschallsignals;
Bestimmen, ob die Standardabweichung der Schallgeschwindigkeit des reflektierten Ultraschallsignals einen vorbestimmten Schwellenwert der Schallgeschwindigkeit des reflektierten Ultraschallsignals überschreitet; und
Identifizieren der Flüssig-Flüssig-Grenzfläche in der Leitung (120), wenn die Standardabweichung der Schallgeschwindigkeit des reflektierten Ultraschallsignals den vorbestimmten Schwellenwert der Schallgeschwindigkeit des reflektierten Ultraschallsignals überschreitet.

10. System nach Anspruch 9, wobei die Leitung (120) eine Ablassleitung ist, die mit einem Chemikalienlagertank (102) gekoppelt ist, wobei die Ablassleitung ein Ablassventil (116) umfasst.

11. System nach Anspruch 10, wobei die Steuerung (132) ferner konfiguriert ist, um ein Signal zum Schließen des Ablassventils (116) zu übertragen, wenn die Flüssig-Flüssig-Grenzfläche in der Leitung (120) identifiziert wird.

12. System nach einem der Ansprüche 9 bis 11, wobei die erste Flüssigkeit ein erstes spezifisches Gewicht aufweist und die zweite Flüssigkeit ein zweites spezifisches Gewicht aufweist, das sich von dem ersten spezifischen Gewicht unterscheidet.

13. System nach Anspruch 12, wobei die erste Flüssigkeit Öl ist und die zweite Flüssigkeit Wasser ist.

## Revendications

1. Procédé d'identification d'une interface liquide/liquide (FI) entre un premier liquide (F1) et un second liquide (F2) dans un conduit (120) à travers lequel au moins le premier liquide (F1) et le second liquide (F2) sont configurés pour passer, le conduit (120) ayant un débitmètre ultrasonore (118) couplé à celui-ci, le procédé comprenant :
la transmission, par l'intermédiaire du débitmètre ultrasonore (118), d'un signal acoustique dans le conduit (120) ;
la réception, par l'intermédiaire du débitmètre ultrasonore (118), d'un signal acoustique réfléchi ;
la détermination, par l'intermédiaire du contrôleur (132), d'une amplitude du signal acoustique réfléchi ;
**caractérisé par** le calcul d'un écart type de l'amplitude du signal acoustique réfléchi mesuré au niveau de divers points temporels ;
la détermination de savoir si l'écart type de l'amplitude du signal acoustique réfléchi dépasse un seuil prédéterminé de l'amplitude du signal acoustique réfléchi ;
l'identification de l'interface liquide/liquide dans le conduit (120) lorsque l'écart type de l'amplitude du signal acoustique réfléchi dépasse le seuil prédéterminé de l'amplitude du signal acoustique réfléchi, dans lequel l'interface liquide/liquide (FI) est identifiée indépendamment de la température ;
la détermination, par l'intermédiaire du contrôleur (132), d'une vitesse de son du signal acoustique réfléchi en fonction du temps de transit du signal acoustique réfléchi ;
le calcul d'un écart type de la vitesse de son du signal acoustique réfléchi ;
la détermination de savoir si l'écart type de la vitesse de son du signal acoustique réfléchi dépasse un seuil prédéterminé de la vitesse de son du signal acoustique réfléchi ; et
l'identification de l'interface liquide/liquide dans le conduit (120) lorsque l'écart type de la vitesse de son du signal acoustique réfléchi dépasse le seuil prédéterminé de la vitesse de son du signal acoustique réfléchi.

2. Procédé selon la revendication 1, dans lequel le premier liquide (F1) a une première gravité spécifique et le second liquide (F2) a une seconde gravité spécifique qui est différente de la première gravité spécifique.

3. Procédé selon la revendication 2, dans lequel le premier liquide (F1) est de l'huile et le second liquide (F2) est de l'eau.

4. Procédé selon la revendication 2, dans lequel le premier liquide est une première huile et le second liquide est une seconde huile.

5. Procédé selon l'une quelconque revendication précédente, comprenant en outre la transmission d'un signal pour indiquer l'identification de l'interface liquide/liquide.

6. Procédé selon l'une quelconque revendication précédente, comprenant en outre la transmission du signal acoustique lorsqu'un liquide s'écoule dans le conduit (120).

7. Procédé selon l'une quelconque revendication précédente, dans lequel le conduit est une conduite de drainage d'un réservoir de stockage chimique (120) et dans lequel le procédé comprend en outre la transmission d'un signal de contrôle pour fermer la conduite de drainage lorsque l'interface liquide/liquide est identifiée.

8. Procédé selon l'une quelconque revendication précédente, comprenant en outre la détermination, par l'intermédiaire du contrôleur (132), d'une vitesse de son du signal acoustique réfléchi en fonction de l'amplitude et du temps de transit du signal acoustique réfléchi.

9. Système d'identification d'une interface liquide/liquide (FI) entre un premier liquide (F1) et un second liquide (F2) dans un conduit (120) à travers lequel au moins le premier liquide (F1) et le second liquide (F2) sont configurés pour passer, comprenant :
un débitmètre ultrasonore (118) couplé au conduit (120), le débitmètre comprenant :
au moins un transducteur ultrasonore (124) couplé au conduit (120) et configuré pour transmettre un signal ultrasonore dans le conduit (120) et recevoir un signal ultrasonore réfléchi ; et
un contrôleur (132) couplé à l'au moins un transducteur à ultrasons (124) et configuré pour :
déterminer une amplitude du signal ultrasonore réfléchi ;
**caractérisé par** le contrôleur (132) étant configuré pour :
calculer un écart type de l'amplitude du signal ultrasonore réfléchi mesuré au niveau de divers points ;
déterminer si l'écart type de l'amplitude du signal ultrasonore réfléchi dépasse un seuil prédéterminé de l'amplitude du signal ultrasonore réfléchi ;
identifier l'interface liquide/liquide dans le conduit (120) lorsque l'écart type de l'amplitude du signal ultrasonore réfléchi dépasse le seuil prédéterminé de l'amplitude du signal ultrasonore réfléchi, dans lequel l'interface liquide/liquide (FI) est identifiée indépendamment de la température ;
déterminer, par l'intermédiaire du contrôleur, une vitesse de son du signal ultrasonore réfléchi en fonction du temps de transit du signal ultrasonore réfléchi ;
calculer un écart type de la vitesse de son du signal ultrasonore réfléchi ;
déterminer si l'écart type de la vitesse de son du signal ultrasonore réfléchi dépasse un seuil prédéterminé de la vitesse de son du signal ultrasonore réfléchi ; et
identifier l'interface liquide/liquide dans le conduit (120) lorsque l'écart type de la vitesse de son du signal ultrasonore réfléchi dépasse le seuil prédéterminé de la vitesse de son du signal ultrasonore réfléchi.

10. Système selon la revendication 9, dans lequel le conduit (120) est une conduite de drainage couplée à un réservoir de stockage chimique (102), la conduite de drainage comprenant une soupape de drainage (116).

11. Système selon la revendication 10, dans lequel le contrôleur (132) est en outre configuré pour transmettre un signal pour fermer la soupape de drainage (116) lorsque l'interface liquide/liquide est identifiée dans le conduit (120).

12. Système selon l'une quelconque des revendications 9 à 11, dans lequel le premier liquide a une première gravité spécifique et le second liquide a une seconde gravité spécifique différente de la première gravité spécifique.

13. Système selon la revendication 12, dans lequel le premier liquide est de l'huile et le second liquide est de l'eau.
